**Europäisches Patentamt**

**European Patent Office**

**Office européen des brevets**

(19)

(11) Veröffentlichungsnummer: **0 271 707**
**B1**

(12) # EUROPÄISCHE PATENTSCHRIFT

(45) Veröffentlichungstag der Patentschrift:
05.09.90

(21) Anmeldenummer: 87116476.0

(22) Anmeldetag: 07.11.87

(51) Int. Cl.⁵: **C07D 301/14**

(54) Verfahren zur kontinuierlichen Durchführung von Epoxidationen.

(30) Priorität: 18.12.86 DE 3643207

(43) Veröffentlichungstag der Anmeldung:
22.06.88 Patentblatt 88/25

(45) Bekanntmachung des Hinweises auf die Patenterteilung:
05.09.90 Patentblatt 90/36

(84) Benannte Vertragsstaaten:
AT BE CH DE FR GB IT LI NL SE

(56) Entgegenhaltungen:
EP-A- 0 056 932
EP-A- 0 061 393
EP-A- 0 183 029
DE-A- 2 747 762
DE-B- 2 519 297
DE-B- 2 519 298
US-A- 3 476 776

(73) Patentinhaber: Degussa Aktiengesellschaft,
Weissfrauenstrasse 9, D-6000 Frankfurt am Main 1(DE)

(72) Erfinder: Siegmeier, Rainer, Dr., Anspacher Strasse 35,
D-6380 Bad Homburg(DE)
Erfinder: Hofen, Willi, Südring 54,
D-6458 Rodenbach 1(DE)
Erfinder: Prescher, Günter, Dr., Liesingstrasse 2,
D-6450 Hanau 9(DE)
Erfinder: Maurer, Helmut, In den Steinäcker 5,
D-6458 Rodenbach 1(DE)

## Beschreibung

Die vorliegende Erfindung betrifft eine Verbesserung bei der kontinuierlichen Epoxydierung von Olefinen mit Percarbonsäuren.

Die bei einer derartigen Epoxydierung anfallenden Oxirane sind interessante Zwischenprodukte, z.B. bei der Synthese von Diolen, Polyäthern oder -urethanen, sowie bei der Herstellung von Lacken.

Die Epoxydation von Olefinen mit Percarbonsäuren ist an sich schon lange bekannt, siehe D. Swern "Organic Peroxides", Wiley Interscience, 1971, Vol II, S. 360 ff. Die dabei verwendeten Percarbonsäuren können - soweit sie wasserlöslich sind - sowohl als wäßrige Lösungen als auch in organischen, praktisch wasserfreien Lösungsmitteln eingesetzt werden, siehe z.B. loc. cit., Seite 375. Dispersionen nichtwasserlöslicher Percarbonsäuren in Wasser werden i.a. nicht verwendet.

Häufig in der Technik eingesetzte Percarbonsäuren sind solche mit 2 - 4 Kohlenstoffatomen, wie Peressigsäure, Perpropionsäure, die Perbuttersäuren.

Die in situ sich bildende Perameisensäure dagegen wird wegen der erhöhten Korrosionsgefahr selbst gegenüber Edelstählen - und ihre dadurch hervorgerufene Zersetzung - siehe DE-AS 25 19 298 - weniger gern als die höhermolekularen Percarbonsäuren genommen.

Auch aromatische Percarbonsäuren, wie z.B. Perbenzoesäure oder Monoperphthalsäure, wurden schon zu Olefinepoxydierungen eingesetzt, siehe DE-OS 23 12 281. Aus ökonomischen Gründen ist eine technische Verwendung dieser Percarbonsäuren nicht sinnvoll.

Unter den Sammelbegriff "Olefine" fielen acyclische und cyclische aliphatische Kohlenwasserstoffe verschiedener Kettenlängen mit einer oder mehreren reaktiven Doppelbindungen.

Die Olefine wurden auch als Schnitte, d.h. untereinander gemischt, eingesetzt. Dies galt vor allem für Olefine ab $C_{12}$ Auch substituierte Olefine, z.B. halogenierte Olefine, wurden schon mit Percarbonsäuren epoxydiert, siehe DE-OS 27 34 243.

Die Epoxydierungen wurden sowohl diskontinuierlich als auch kontinuierlich durchgeführt. Da die Olefine bei kontinuierlicher Fahrweise gegenüber der Percarbonsäure im allgemeinen in einem mehr oder weniger großen Überschuß eingesetzt wurden, wurde dieser Überschuß im Kreislauf in die Epoxydationsstufe wieder zurückgeführt, siehe DE-OS 31 01 037 , DE-OS 34 42 937.

Käufliche Olefine enthalten trotz vorher durchgeführter Reinigungsverfahren bekanntlich noch immer einen gewissen Rest an Verunreinigungen. Dieser besteht bei Monoolefinen in erster Linie aus Alkanen, deren Siedepunkte denen der Olefine häufig sehr ähnlich sind. Dieser Restgehalt an Alkanen reichert sich bei Kreislaufverfahren in dem Einsatzolefin, das mit dem rückgeführten Olefin gemischt wird, immer mehr an und belastet damit die Durchführung des gesamten Verfahrens.

Eine destillative Reinigung des rückgeführten Olefins mit Abtrennung der Alkane ist wegen der Ähnlichkeit der Siedepunkte nicht immer möglich.

Auch extraktive Destillationen sind innerhalb eines Epoxydierungsverfahrens wegen des zusätzlichen Aufwandes an verfahrensfremden Lösungsmitteln zu aufwendig.

Die bekannten chemischen Trennverfahren sind nicht anwendbar, da sie z.B. auf der Abtrennung der Diolefine von Monoolefinen, nicht aber auf der Abtrennung von Alkanen, beruhen, siehe ULLMANN, Enzyklopädie der technischen Chemie, 3. Auflage (1958) Band 10, Seite 62/63.

Aufgabe der Erfindung ist es daher, die bei Epoxydationsverfahren in Kreislaufolefinen als Beimengungen vorliegenden inerten Anteile, hauptsächlich Alkane, ohne Verwendung reaktionsfremder Chemikalien auf einem niedrigen Niveau zu halten bzw. zu entfernen.

Es wurde nun gefunden, daß sich diese Aufgabe lösen läßt, wenn man aus dem bei der Epoxydierung im Kreislauf geführten Olefin oder Olefingemisch, das die inerten Verbindungen, hauptsächlich Alkane, enthält, einen Teilstrom entnimmt und ihn mit einer Percarbonsäurelösung epoxydiert, worauf das Gemisch aus neu gebildeten Epoxiden und nicht epoxydierten, inerten Anteilen in bekannter Weise aufgetrennt wird.

Bevorzugt wird man zur Epoxydierung der im Teilstrom enthaltenen Olefine die Percarbonsäure verwenden, die auch im Hauptstrom benutzt wurde.

Im Prinzip können alle bei Epoxydierungsverfahren verwendeten Percarbonsäuren eingesetzt werden; besonders günstig waren aber diejenigen mit 2 - 4 Kohlenstoffatomen, die man bevorzugt in organischen Lösungsmitteln, wie z.B. in aliphatischen, cycloaliphatischen oder aromatischen Kohlenwasserstoffen, wie Pentan, 2-Äthylhexan, Cyclohexan, Methylcyclopentan, oder Benzol, Toluol, Tetralin, oder in Äthern bzw. Estern, wie Tetrahydrofuran, Propionsäureäthylester, Essigsäureäthylester; Essigsäurebutylester, oder in chlorierten Kohlenwasserstoffen, wie Methylenchlorid, Chloroform, Tetrachlorkohlenstoff oder Dichloräthan, Dichlorpropan bzw. Chlorbenzol wie den Dichlorbenzolen einsetzt. Auch Gemische der genannten Lösungsmittel können verwendet werden.

Die Konzentrationen dieser Percarbonsäurelösungen liegen im allgemeinen zwischen 5 bis 50 Gew.-%.

Die erfindungsgemäße Verminderung der nicht epoxidierbaren Anteile im rückgeführten Olefin oder Olefingemisch ist anwendbar bei allen Epoxydierungsverfahren, die mit einem Überschuß an Olefin gegenüber der Percarbonsäure und mit einer Rückführung des nicht umgesetzten Olefins im Kreislauf arbeiten.

Bevorzugt wird man diese Verminderung der nicht epoxidierbaren Anteile so durchführen, daß ein vorgegebenes, niedriges Mengenniveau dieser Anteile in dem Olefinhauptstrom kontinuierlich aufrecht erhalten wird. Die Größe des zu entnehmenden Teilstroms bzw. die Menge der für diesen Zweck einzusetzenden Percarbonsäure richtet sich vorzugsweise nach der sich im Kreislaufstrom einstellenden stationären Konzentration der nicht epoxidierbaren inerten Anteile. Dieses läßt sich durch einen Handversuch leicht feststellen.

Die nach der erfolgten Epoxydierung der Olefine in dem Teilstrom vorliegende Mischung aus neu gebildeten Epoxiden und nicht epoxydierbaren Anteilen wird nun in bekannter Weise aufgetrennt. Durch die Epoxydierung hat sich die Siedepunktsdifferenz zwischen den Epoxiden und Alkanen gegenüber denen der Olefine und Alkane so vergrößert, daß die Auftrennung durch eine übliche Destillation und/oder Desorption möglich wird.

Das erfindungsgemäße Verfahren ist günstig bei der kontinuierlichen Epoxydation von Olefinen mit der Kettenlänge von 3 - 12 Kohlenstoffatomen bzw. der höheren Kettenlänge mit bis zu 20 Kohlenstoffatomen. Die Epoxydierung wird mit Percarbonsäuren mit 2 - 4 Kohlenstoffatomen, die bevorzugt in den auf Seite 4 genannten Lösungsmitteln vorliegen, durchgeführt. Ganz besonders günstig ist die erfindungsgemäße Epoxydation bei Olefinen mit 3 - 8 Kohlenstoffatomen. Die Konzentration der Percarbonsäuren in den genannten Lösungsmitteln ist zwar nicht kritisch, liegt aber bevorzugt bei 10 - 30 Gew.-%.

Sehr günstige Lösungsmittel sind Benzol, Toluol, Cyclohexan, Äthylacetat, Dichlorpropan bzw. Chloroform. Ganz besonders eignet sich das erfindungsgemäße Verfahren zur Epoxydierung von Penten mit benzolischer Perpropionsäure.

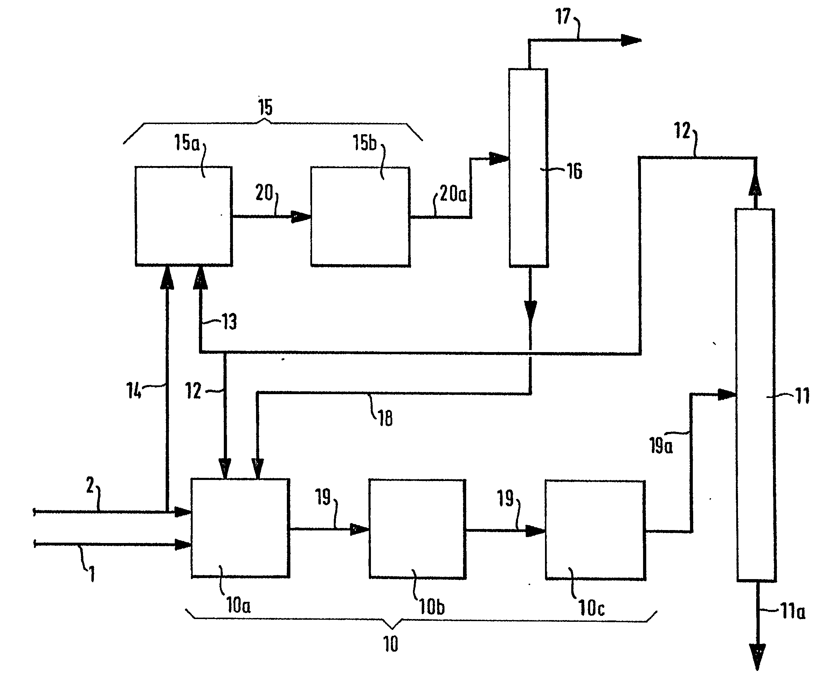

Das erfindungsgemäße Verfahren wird anhand der Abbildung 1 und des Beispiels näher erläutert:

In der schematischen Abbildung 1 bedeuten die Ziffern 10a, 10b und 10c drei hintereinandergeschaltete Reaktoren für die Hauptreaktion 10, nämlich die Epoxydierung von Olefinen mit Percarbonsäuren. Als Reaktoren kommen z.B. Rührkesselkaskaden oder Schaufelreaktoren mit nachgeschaltetem Strömungsrohr in Frage.

Über Leitung 1 wird das jeweilige Olefin bzw. Olefingemisch, über Leitung 2 die Lösung der Percarbonsäure, die entweder wäßrig oder in einem organischen Lösungsmittel sein kann, in das System 10 eingeführt. Die Mischung aus Reaktor 10a, die schon zu einem Teil aus den Reaktionsprodukten besteht, durchläuft über die Leitungsteile 19 die Reaktoren 10b und 10c und tritt praktisch ausreagiert über 19a in eine Aufarbeitungsvorrichtung 11, z.B. eine Destillationsvorrichtung, gegebenenfalls auch kombiniert mit einer Desorptionskolonne, ein, in der das überschüssige Olefin zusammen mit Alkan abgetrennt und über Leitung 12 der Hauptreaktion 10, z.B. dem Reaktor 10a, zugeführt wird. Über Leitung 11a verläßt das Reaktionsprodukt die Aufarbeitungsvorrichtung 11.

Entsprechend dem erfindungsgemäßen Verfahren wird nun aus Leitung 12 ein Teil des überdestillierten Gemisches aus Olefin und Alkan über Leitung 13 entnommen und in ein Aufarbeitungs- oder Vorreaktionssystem 15 eingeführt, das aus zwei Reaktoren 15a und 15b bestehen kann, die hintereinandergeschaltet sind. Auch hier kommen beispielsweise die oben schon genannten Reaktorarten für das System in Frage. In Reaktor 15a tritt ein Zweigstrom 14 der über Leitung 2 einströmenden Percarbonsäurelösung ein;

die Mischung, die zum Teil schon reagiert hat, gelangt über Leitung 20 in den Reaktor 15b und als praktisch ausreagierte Mischung über Leitung 20a in die Aufarbeitungsvorrichtung 16. Diese kann eine Destillations- oder Abstreifkolonne sein, gegebenenfalls kombiniert mit einer Desorptionsvorrichtung. Während die Leichtsieder - d.h. ein geringer Rest an Olefin und das Alkan - über Leitung 17 das System verlassen, strömen die neu gebildeten Epoxide über Leitung 18 wieder der Hauptreaktion 10, z.B. dem Reaktor 10a, zu.

Beispiel:

Einem kontinuierlich geführten Reaktionssystem (Hauptreaktion 10) - siehe Abbildung 1 - bestehend aus zwei Schlaufenreaktoren 10a und 10b und einem Strömungsrohr 10c, werden 209 g/h Frischpenten über Leitung 1 (95,2 % Penten, 4,8 % Pentan) und 848 g/h benzolische Perpropionsäure ( ~ 22 % Persäure) über Leitung 2 zugeführt und dabei in den ersten Reaktor 10a eingespeist.

Von einem Kreislaufstrom 12 (75 % Penten, 25 % Pentan), der nach der Hauptreaktion destillativ in Kolonne 11 aus dem Reaktionsgemisch erzeugt wird, werden 90 g/h Rückpenten ebenfalls dem ersten Reaktor 10a der Hauptreaktion 10 zugeführt.

Der Rest des Kreislaufstroms 12 - ca. 40 g/h - wird über Leitung 13 einem Vorreaktionssystem 15, bestehend aus einem Schlaufenreaktor 15a und einem Strömungsrohr 15b (Gesamtverweilzeit 50 Minuten), mit 268 g/h benzolischer Perpropionsäurelösung mit einem Perpropionsäuregehalt von 22 Gew.-%, die über Leitung 14 vom Hauptstrom 2 der Perpropionsäurelösung abgezweigt wird, so weit umgesetzt, daß der Olefinanteil zu etwa 96 % abreagiert. Die Reaktionsmischung der Vorreaktion 15 wird nachfolgend in einer Strippkolonne 16 bei 300 mbar destillativ von den Leichtsiedern Penten und Pentan befreit. Man erhält ca. 11 g/h Leichtsieder, davon ca. 10 g/h Pentan. Der Ablauf der Strippkolonne 16 (297/h) strömt dem ersten Schlaufenreaktor 10a der Hauptreaktion über Leitung 18 zu. Er enthält die reaktiv aus dem Teilstrom des Rückpentens gewonnenen Anteile an Epoxiden.

Die gesamte Reaktionsmasse (1444 g/h) durchströmt die Hauptreaktion 10a - 10c bei einer Verweilzeit von ca. 60 min und wird nachfolgend destillativ aufgearbeitet. Dabei entsteht nun wieder das Kreislaufolefin (130 g/h), welches in beschriebener Weise als Teilströme der Vorreaktion 15 der Hauptreaktion 10 zugeführt wird. Auf Grund des in der Vorreaktion 15 abgezweigten Teilstromes und der nach Abreaktion des Olefinanteils nun ermöglichten konzentrierten Ausschleusung des Pentans befindet sich die Pentankonzentration im gesamten Kreisstrom im stationären Gleichgewicht (ca. 25 %). Alle Prozentangaben sind Gewichtsprozente.

**Patentansprüche**

1. Kontinuierliches Verfahren zur Epoxydierung von Olefinen mit Percarbonsäuren, die in wäßriger Lösung oder in organischen Lösungsmitteln vorliegen, und bei dem die Olefine im Überschuß gegenüber der Percarbonsäure eingesetzt werden und der nicht umgesetzte Olefinanteil im Kreislauf geführt wird, dadurch gekennzeichnet, daß man aus dem bei der Epoxydierung im Kreislauf geführten Olefin oder Olefingemisch einen Teilstrom entnimmt, ihn mit einer Percarbonsäurelösung epoxydiert und das Gemisch aus neugebildeten Epoxiden und nicht epoxydierbaren Anteilen in bekannter Weise auftrennt.

2. Verfahren nach Anspruch 1, dadurch gekennzeichnet, daß man zur Epoxydierung der im Teilstrom enthaltenen Olefine die Percarbonsäure verwendet, die auch im Hauptstrom benutzt wird.

3. Verfahren nach Anspruch 1 und 2, dadurch gekennzeichnet, daß man Percarbonsäuren mit 2–4 Kohlenstoffatomen einsetzt.

4. Verfahren nach Anspruch 1 bis 3, dadurch gekennzeichnet, daß man Percarbonsäure in benzolischer Lösung verwendet.

5. Verfahren nach Anspruch 1 bis 4, dadurch gekennzeichnet,daß man die Epoxydation des Teilstroms mit benzolischer Perpropionsäure bei der Epoxydierung von Penten mit benzolischer Perpropionsäure durchführt.

## Claims

1. Continuous process for the epoxidation of olefins with percarboxylic acids present in aqueous solution or in organic solvents, in which the olefins are used in excess over the percarboxylic acid and the unreacted portion of olefin is circulated, characterised in that a partial stream is removed from the olefin or olefin mixture circulated in the process of epoxidation, the partial stream is epoxidised with a percarboxylic acid solution and the mixture of newly formed epoxides and non-epoxidisable components is separated in known manner.

2. Process according io Claim 1, characterised in that the percarboxylic acid used in the main stream is used for epoxidising the olefins contained in the partial stream.

3. Process according to Claims 1 and 2, characterised in that percarboxylic acids having 2–4 carbon atoms are used.

4. Process according to Claims 1 to 3, characterised in that percarboxylic acid is used in a benzene solution.

5. Process according to Claims 1 to 4, characterised in that epoxidation of the partial stream is carried out with perpropionic acid in benzene during the process of epoxidation of pentene with perpropionic acid in benzene.

## Revendications

1. Procédé d'époxydation en continu d'oléfine par les peracides carboxyliques, qui sont en solution aqueuse ou en solvants organiques, et dans lequel on utilise l'oléfine en excès par rapport au peracide carboxylique, et dans lequel on recycle la fraction d'oléfine qui n'a pas réagi, caractérisé en ce qu'on prélève un courant partiel de l'oléfine ou du mélange d'oléfine impliqué dans le circuit d'époxydation, on l'époxyde par une solution de peracide carboxylique et on fractionne de manière connue les époxydes qui viennent d'être formés et les fractions non époxydables.

2. Procédé selon la revendication 1, caractérisé en ce qu'on utilise pour époxyder les oléfines du courant partiel le peracide carboxylique qui est aussi utilisé dans le courant principal.

3. Procédé selon les revendications 1 et 2, caractérisé en ce qu'on utilise des peracides carboxyliques avec 2–4 atomes de carbone.

4. Procédé selon les revendications 1 à 3, caractérisé en ce qu'on utilise du peracide carboxylique en solution benzénique.

5. Procédé selon les revendications 1 à 4, caractérisé en ce qu'on réalise l'époxydation du courant partiel avec du peracide propionique en solution benzénique en époxydant du pentène avec du peracide propionique en solution benzénique.

EP 0 271 707 B1

17

15

15a 15b

20 20a 16

13

14 12

18

12

11

19a

2

19 19 11

1

10a 10b 10c

10

11a